(19) Europäisches Patentamt
European Patent Office
Office européen des brevets

(11) EP 2 355 753 B1

(12) EUROPÄISCHE PATENTSCHRIFT

(45) Veröffentlichungstag und Bekanntmachung des Hinweises auf die Patenterteilung:
**13.02.2013 Patentblatt 2013/07**

(21) Anmeldenummer: **09804000.9**

(22) Anmeldetag: **01.12.2009**

(51) Int Cl.:
**A61F 2/50** (2006.01)      **A61F 2/66** (2006.01)

(86) Internationale Anmeldenummer:
**PCT/DE2009/001703**

(87) Internationale Veröffentlichungsnummer:
**WO 2010/063274 (10.06.2010 Gazette 2010/23)**

(54) **VERFAHREN ZUR HERSTELLUNG EINES KÜNSTLICHEN FUSSES**

METHOD OF MANUFACTURING A PROSTHETIC FOOT

PROCÉDÉ DE FABRICATION D'UNE PIED PROTHETIQUE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **02.12.2008 DE 102008060177**

(43) Veröffentlichungstag der Anmeldung:
**17.08.2011 Patentblatt 2011/33**

(73) Patentinhaber: **Otto Bock Healthcare GmbH
37115 Duderstadt (DE)**

(72) Erfinder:
• **ZARLING, Sven
37115 Duderstadt (DE)**
• **KARSTENS, Martin
37073 Göttingen (DE)**

(74) Vertreter: **Lins, Edgar et al
Gramm, Lins & Partner GbR
Theodor-Heuss-Strasse 1
38122 Braunschweig (DE)**

(56) Entgegenhaltungen:
**WO-A1-2005/048887      GB-A- 2 433 443
US-A1- 2003 144 745**

EP 2 355 753 B1

**Beschreibung**

Die Erfindung betrifft ein Verfahren zur Herstellung eines künstlichen Fußes der in einer Längsrichtung eine mediale Ebene aufweist, in der eine Nenn-Fußlänge einen Abstand von einer Ferse und einer Fußspitze eines von dem künstlichen Fuß ersetzten natürlichen Fußes definiert ist, und der mit einem oberseitigen Anschlussstück zur momentensteifen Verbindung eines sich im Wesentlichen über die Fußlänge erstreckenden Fußeinsatzes ausgebildet ist, der - über die Länge gesehen - an zwei Auflageflächen aufliegt, von denen sich eine erste fersenseitige Auflagefläche im Fersenbereich und eine zweite ballenseitige Auflagefläche im Ballenbereich befindet und der so ausgebildet ist, dass das Anschlussteil über federnde Verbindungen mit den Auflageflächen des Fußteils verbunden ist, wobei die balligen Auflageflächen zu einer angenähert linienförmigen Auflagefläche auf einem Untergrund ausgebildet sind

Künstliche Füße dieser Art sind in zahlreichen Ausführungsformen bekannt. Sie sind in verschiedenster Hinsicht optimiert worden. Eine wesentliche Funktion eines künstlichen Fußes besteht in der Gewährleistung eines möglichst natürlichen Gangbildes im Gehzyklus, wobei dem Prothesenträger ein sicheres Ganggefühl vermittelt werden soll. Darüber hinaus soll der künstliche Fuß ein möglichst stabiles und als sicher empfundenes Stehen ermöglichen.

Aus US 2003/0144745 A1 ist ein künstlicher Fuß bekannt, bei dem das Anschlussstück mit einem sich über die Fußlänge erstreckenden Fußeinsatz in Form eines Sohlenstücks durch geeignete Verbindungsmittel verbunden ist. Das Anschlussstück ist gebogen so ausgebildet, dass es eine federnde Verbindung zwischen einem Anschlussteil zu einem Unterschenkelpylon einerseits und dem Fußeinsatz andererseits herstellt. Der gebogene Abschnitt des Anschlussstücks kann durch ein Zusatzstück verstärkt werden, sodass die Federhärte der federnden Verbindung variierbar ist. Die Teile des künstlichen Fußes können aus Kunststofflaminaten gebildet sein.

GB 2 433 443 A offenbart eine Prothese zur Gangkorrektur, die im Wesentlichen aus einem kosmetisch geformten Teil besteht, das einen Fuß und einen Unterschenkelansatz nachbildet. Das Teil ist als Hohlkörper ausgebildet, wobei der Hohlraum an den durch Verkrüppelung oder Amputation unnormal ausgebildeten Fuß angepasst ist. In den Sohlenabschnitt des Fußteils ist eine gebogene, federnde Karbonfaserplatte eingelegt, die die Abrolleigenschaften der Fußprothese bestimmt. Eine besondere Unterstützung beim Stehen bildet diese Fußprothese nicht.

Es hat sich gezeigt, dass künstliche Füße für den Gangzyklus als besonders geeignet herstellbar sind, die jedoch dann ein unsicheres Gefühl beim Stehen auf dem künstlichen Fuß vermitteln. Wird der künstliche Fuß hingegen für das Stehen optimiert, ergibt sich ein beeinträchtigtes Gangbild in Verbindung mit einem unsicheren Gefühl beim Gehen.

Der vorliegenden Erfindung liegt daher die Aufgabe zugrunde, ein Verfahren zur Herstellung eines künstlichen Fußes anzugeben, mit dem sowohl ein gutes Ganggefühl als auch ein sicheres Gefühl beim Stehen durch den künstlichen Fuß vermittelt wird.

Die vorliegende Erfindung geht von der Erkenntnis aus, dass bei einem natürlichen Bein mit einem natürlichen Fuß der durch die Bodenreaktionskräfte gebildete resultierende Kraftvektor (Körpervektor) in einigem Abstand vor dem als Lasteinleitungspunkt vom Unterschenkel zum Fuß wirkenden Knöchelgelenk liegt. Es ist daher grundsätzlich bekannt, eine entsprechende Konstruktion auch für künstliche Füße vorzusehen also den Körpervektor, der sich aus einer vektoriellen Summe von Bodenreaktionskräften ergibt, vor einer Lasteinleitungs-Mittenachse, über die Gewichtskräfte in den künstlichen Fuß eingeleitet werden, anzuordnen. Da somit die Lasteinleitung in den künstlichen Fuß nicht in einer durch die Gelenkachse verlaufenden Linie erfolgt, ergibt sich über den horizontal liegenden Abstandshebel ein Drehmoment in den Gelenken beim Übergang von dem unbelasteten Fuß in den mit dem Gewicht belasteten Fuß.

Zur Lösung der oben erwähnten Aufgabe ist erfindungsgemäß ein Verfahren zur Herstellung eines künstlichen Fußes der eingangs erwähnten Art dadurch gekennzeichnet, dass

a) Positionierung eines Körpervektors als resultierenden Kraftvektor der an den Auflageflächen entstehenden Bodenreaktionskräften im ruhenden Stand in einem Bereich zwischen 40 % und 48 % der Nenn-Fußlänge, von der Ferse aus gemessen,

b) Belastung des Fußes mit Gewichtsbelastungen zwischen 25 % des minimal zulässigen und 80 % des maximal zulässigen Körpergewichts und

c) Abstimmung der federnden Verbindungen zwischen dem Anschlussteil und den Auflageflächen derart, dass sich bei der Belastung gemäß Verfahrensschritt b) der Körpervektor um weniger als $\pm$ 4 % der Nenn-Fußlänge verschiebt.

Der vorliegenden Erfindung liegt der Gedanke zugrunde, dass ein sicheres Gefühl beim Stehen des Prothesenträgers voraussetzt, dass sich der resultierende Kraftvektor (Körpervektor) aus den an den Auflageflächen entstehenden Bodenreaktionskräften bei einer variierenden Belastung des künstlichen Fußes nicht wesentlich in seiner Lage in Längsrichtung verändern sollte. Eine derartige Veränderung des Körpervektors ergibt sich bei den bekannten Aufbauten von künstlichen Füßen. Erfindungsgemäß sind die federnden Verbindungen zwischen dem Anschlussteil und den Auflageflächen des Fußteils so aufeinander abgestimmt, dass sich der Körpervektor bei variierender Belastung des künstlichen Fußes in Längsrichtung im Wesentlichen nicht verschiebt, d. h. eine Variation von ± 4 %, vorzugsweise von ± 2 % nicht überschreitet. Ferner ist für die vorliegende Erfindung von Bedeutung, dass sich der Körpervektor innerhalb eines vorgegebenen Bereichs befindet, nämlich zwischen 40 und 48 % der Nenn-Fußlänge, von der Ferse aus gemessen, wobei die optimale Einstellung bei 44 %, ggf. ± 1 %, liegt.

Die Ausbildung der balligen Auflageflächen zu einer angenähert linienförmigen Auflagefläche auf einem Untergrund hat den Sinn, definierte Auflageflächen zu gewährleisten, die bei einer Gewichtsverlagerung in der Sagittalebene zu einer oder nur zu einer sehr geringen Veränderung der Position der Auflagefläche in der Längsrichtung (in der Sagitttalebene) führen. Die linienförmige Auflagefläche kann daher auch durch punktförmige Auflageflächen gebildet sein, die eine resultierende Linie bilden.

Es hat sich gezeigt, dass ein erfindungsgemäß hergestellter künstlicher Fuß ein sicheres Gefühl beim Stehen vermittelt, das dem Gefühl beim Stehen auf dem gesunden Fuß entspricht. Darüber hinaus lässt sich der erfindungsgemäß hergestellte künstliche Fuß ohne weiteres für ein natürliches und sicheres Gefühl beim Gehen des Prothesenträgers auslegen.

Insgesamt ist es bevorzugt, wenn der aus den Bodenreaktionskräften entstehende Körpervektor zwischen 42 und 46 % der Nenn-Fußlänge verbleibt.

Ein ruhender Stand ergibt sich, wenn der Körper aufgerichtet und die Körperlast gleichmäßig auf beide Füße verteilt ist, sodass die Muskulatur nur geringfügig beansprucht wird. Messungen des Schwerelots im ruhenden Stand haben ergeben, dass der Körpervektor ausgehend von der Mitte des Kopfes (Gehörgang) 1 cm anterior des Wirbelkörpers L4 bis zu einem Punkt 1,5 bis 5 cm vor dem oberen Sprunggelenk verläuft. Der ruhende Stand ist nicht völlig stabil, da es leichte Ausgleichsbewegungen gibt, die etwa 4 bis 6 x pro Sekunde mit Schwankungen von bis zu 5 mm lateral und bis zu etwa 8 mm nach anterior und posterior erfolgen.

Der erfindungsgemäß hergestellte künstliche Fuß lässt sich mit mehreren Konstruktionen realisieren.

In einer ersten Ausführungsform kann der Fußeinsatz einen Kern aufweisen und mit den Auflageflächen über elastische Stücke verbunden sein. Der Kern kann dabei unelastisch ausgebildet sein, kann aber auch eine vorbestimmte Elastizität aufweisen, die sich insbesondere über die Länge des Fußteils zum Zehenbereich hin verringert.

Die elastischen Stücke können aus elastisch komprimierbarem Material bestehen und die balligen Auflageflächen bilden. Alternativ ist es möglich, zwischen den Auflageflächen und dem Kern Federn als elastische Stücke einzusetzen.

In einer Weiterbildung dieser Ausführungsform kann das Anschlussteil mit dem Kern über ein Gelenk verbunden sein, das bei Belastung aus dem ruhenden Stand heraus eine elastisch bedämpfte Drehung des Anschlussteils relativ zur fersenseitigen Auflagefläche ermöglicht. Diese Ausführungsform ermöglicht eine erweiterte Plantarflexion des Fußes. Vorzugsweise wird diese Plantarflexion durch ein flexibles, vorzugsweise unelastisches, Begrenzungsmittel in Richtung Expansion der Feder begrenzt.

Es ist ferner möglich, den Fußeinsatz weitgehend oder vollständig ohne starren Kern als eine Federkombination auszubilden, die eine zur fersenseitigen Auflagefläche verlaufende Feder und eine zur ballenseitigen Auflagefläche verlaufende Feder aufweist, wobei die Federn am Anschlussteil miteinander verbunden werden. In einer weiteren Ausbildung dieses Konstruktionsprinzips können die Federn bodenseitig durch eine sich über die Länge des Fußteils erstreckende weitere Feder miteinander verbunden werden.

Vorzugsweise werden die Federn der Federkombination durch Blattfedern gebildet.

In allen Fällen ist es für die vorliegende Erfindung wesentlich, dass die Elastizitäten zwischen den Auflageflächen und dem Anschlussteil so aufeinander abgestimmt werden, dass die erfindungsgemäße Konstanz der Lage des durch die Bodenreaktionskräfte definierten Schwerpunkts innerhalb des angegebenen Bereichs gewährleistet ist.

Die Erfindung soll im Folgenden anhand von in der Zeichnung dargestellten Ausführungsbeispielen näher erläutert werden. Es zeigen:

Figur 1     eine schematische Seitenansicht eines erfindungsgemäß hergestellten künstlichen Fußes;

Figur 2     eine Darstellung einer Schnittebene in der Längs-Mittenachse des Fußes;

Figur 3     eine schematische Darstellung der Auswirkung unterschiedlicher Absatzhöhen auf dem erfindungsgemäß hergestellten künstlichen Fuß;

Figur 4     eine schematische Darstellung der federnden Verbindungen zwischen den Auflageflächen und dem Anschlussteil des Fußes;

Figur 5     eine schematische Darstellung eines Experiments zur Simulation des Stehens bei unterschiedlichen Gewichtsbelastungen;

Figur 6     eine schematische Darstellung eines geringfügig modifizierten Experiments gemäß Figur 3;

Figur 7     eine Darstellung einer Schwerpunktslinie eines erfindungsgemäß hergestellten künstlichen Fußes;

Figur 8     eine schematische Darstellung einer möglichen Konstruktion eines erfindungsgemäß hergestellten künstlichen Fußes nach einer ersten Ausführungsform;

Figur 9    eine schematische Darstellung einer möglichen Konstruktion eines erfindungsgemäß hergestellten künstlichen Fußes nach einer zweiten Ausführungsform;

Figur 10   eine schematische Darstellung einer möglichen Konstruktion eines erfindungsgemäß hergestellten künstlichen Fußes nach einer dritten Ausführungsform;

Figur 11   eine schematische Darstellung einer möglichen Konstruktion eines erfindungsgemäß hergestellten künstlichen Fußes nach einer vierten Ausführungsform;

Figur 12   eine schematische Darstellung einer möglichen Konstruktion eines erfindungsgemäß hergestellten künstlichen Fußes nach einer fünften Ausführungsform;

Figur 13   eine schematische Darstellung einer möglichen Konstruktion eines erfindungsgemäß hergestellten künstlichen Fußes nach einer sechsten Ausführungsform;

Figur 14   eine schematische Darstellung einer möglichen Konstruktion eines erfindungsgemäß hergestellten künstlichen Fußes nach einer siebten Ausführungsform.

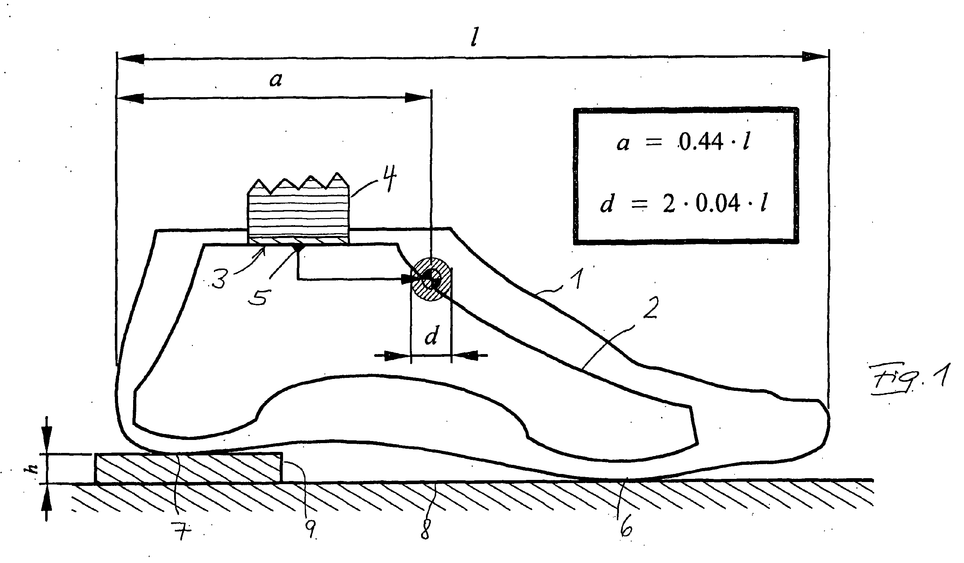

Figur 1 verdeutlicht den prinzipiellen Aufbau eines künstlichen Fußes, der aus einer die Form und das Aussehen eines natürlichen Fußes imitierenden kosmetischen Hülle 1 und aus einem Fußeinsatz 2 gebildet wird. Das Fußteil 2 weist einen Anschluss 3 auf, über die ein Unterschenkel-Prothesenteil 4 momentensteif an den künstlichen Fuß anschließbar ist. Demgemäß wird das Gewicht des Patienten über den Anschluss 3 in den künstlichen Fuß eingeleitet. Schematisch ist ein Krafteinleitungspunkt vom Unterschenkel-Prothesenteil 4 in den künstlichen Fuß eingezeichnet.

Über das Fußteil 2 wird das Gewicht auf zwei ballig ausgebildete Auflageflächen 6, 7 verteilt, von denen eine ballenseitige Auflagefläche 6 etwa in der Höhe des Fußballens und eine fersenseitige Auflagefläche 7 etwa in der Höhe der Ferse des Fußes ausgebildet ist. Die ballenseitige Auflagefläche 6 ist zur Auflage auf einem Untergrund 8 vorgesehen, während die fersenseitige Auflagefläche 7 auf dem Untergrund 8 über eine Standard-Absatzhöhe 9 aufliegen soll.

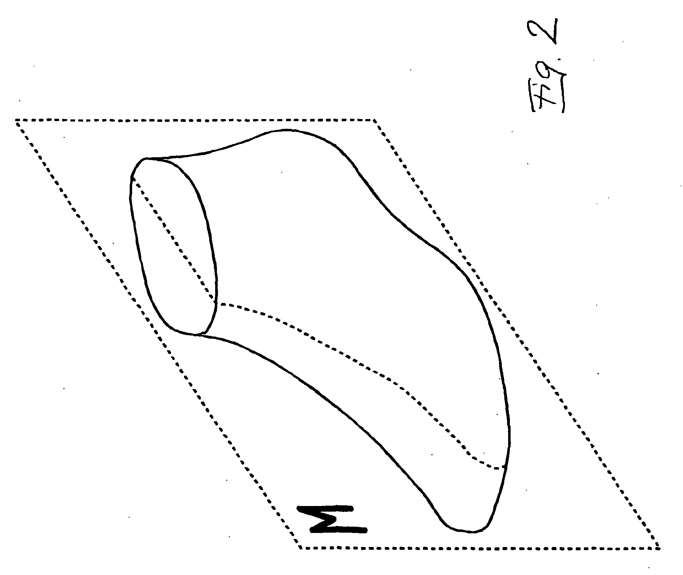

Der künstliche Fuß ist mit einem Aufbau-Bezugspunkt A konstruiert, durch den der konstruktive Körpervektor der Prothese bei unbelastetem Fuß hindurchlaufen soll. Der künstliche Fuß hat eine mediale Ebene M, die in Figur 2 verdeutlicht ist. Sie steht vertikal und enthält die Mittenachse in Längsrichtung und den Aufbau-Bezugspunkt A.

Der künstliche Fuß hat einen Nennlänge I, die der Länge des durch den künstlichen Fuß zu ersetzenden natürlichen Fußes entspricht.

Die Nennlänge I verläuft in der medialen Ebene M von der Projektion der Zehenspitze des großes Zehs bis zur Projektion der Ferse.

Bei dem erfindungsgemäßen Fuß liegt der Aufbau-Bezugspunkt A bei einem Abstand von 0,44 x I vom hinteren Fersenende. Wird der Fuß vertikal belastet, soll sich der aus den Bodenreaktionskräften an den Auflageflächen 6, 7 resultierende Kraftvektor innerhalb eines Bereichs d befinden, der einen Radius von 0,04 x I um den Aufbau-Bezugspunkt A aufweist, also einen Durchmesser von 2 x 0,04 x I hat.

Ein künstlicher Fuß wird für einen Gewichtsbereich eines Patienten ausgelegt, der von einem unteren Nenngewicht mU bis zu einem oberen Nenngewicht mO reicht. Der durch die Bodenreaktionskräfte an den Auflageflächen 6, 7 resultierende Kraftvektor soll innerhalb des Toleranzbereichs d bei einer Gewichtsbelastung von 0,25 x mU bis 0,8 mO bleiben.

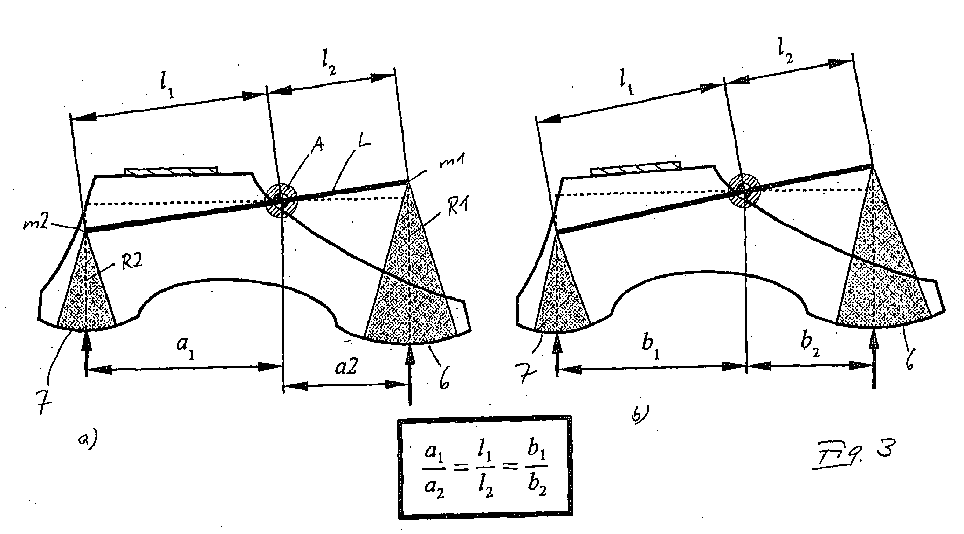

Figur 3 verdeutlicht die Bedeutung der balligen Ausführung der Auflageflächen 6, 7 und deren Funktion bei sich ändernden Absatzhöhen.

Figur 3a zeigt den künstlichen Fuß in seiner Stellung mit einer Nenn-Absatzhöhe. Die balligen Auflageflächen 6, 7 sind als Kreisbogenabschnitte in der medialen Ebene M ausgebildet. Die Radien R1, R2, sind dabei so gewählt, dass eine Verbindungslinie L durch die Mittelpunkte m1, m2 der Kreisbögen der Auflageflächen 6, 7 durch den Aufbau-Bezugspunkt A hindurchlaufen. Bei dieser Ausbildung ändert sich das Verhältnis der Strecken a1, a2 von den Auflagelinien 6, 7 zu Projektion des Aufbau-Bezugspunkts A auf dem Untergrund 8 nicht, wie anhand der Figur 3b abzulesen ist. In Figur 3b ist der künstliche Fuß ohne Absatzhöhe dargestellt. Dadurch verschieben sich die Auflagelinien der Auflageflächen 6, 7, woraus sich die Strecken b1 und b2 ergeben. Wenn die oben erwähnte Bedingung für die Balligkeit der Flächen 6, 7 in Längsrichtung eingehalten werden, ergibt sich, dass die Verhältnisse der Strecken a1 zu a2 und b1 zu b2 gleich sind. Das Verhältnis entspricht dem unverändert bleibenden Abstand der Mittelpunkte m1 und m2 zum Aufbau-Bezugspunkt A entlang der Verbindungslinie L (l1:l2).

Die Wirkung des erfindungsgemäßen Fußes beruht auf der Abstimmung der federnden Verbindung zwischen den Auflagelinien der Auflageflächen 6, 7 und dem Aufbau-Bezugspunkt A. Dieses Verhältnis bleibt somit gleich, sodass die für die Nenn-Absatzhöhe (Figur 3a) eingestellte Abstimmung der Federwege auch bei einer sich ändernden Absatzhöhe (Figur 3b) erhalten bleibt.

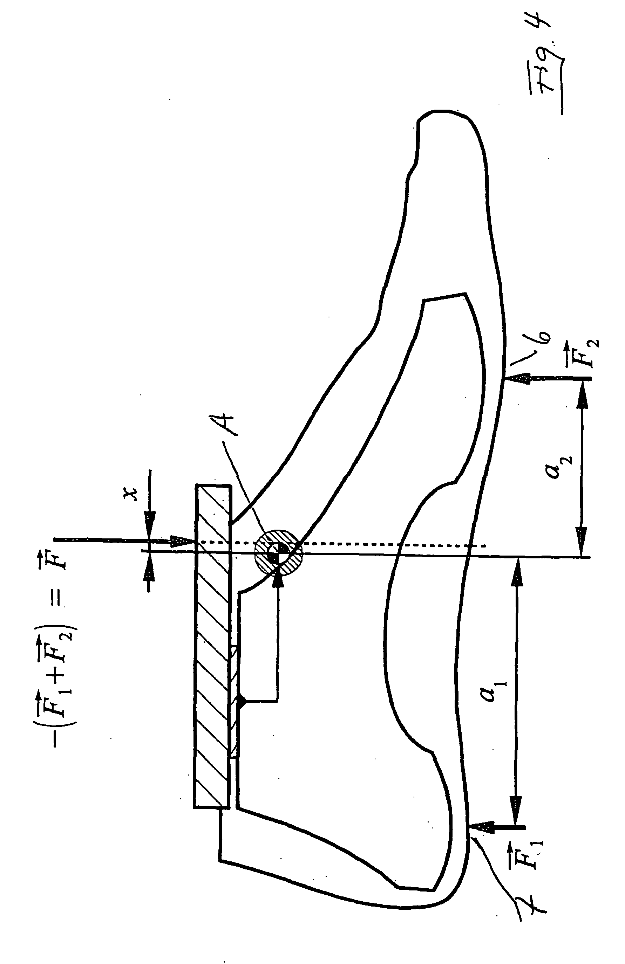

Figur 4 verdeutlicht die Anpassung der federnden Verbindungen zwischen den Auflagelinien der Auflageflächen 6, 7 und dem Anschlussteil 4 bzw. dem Aufbau-Bezugspunkt A. Beim unten näher erläuterten "simulierten Stehen" verläuft der Körpervektor durch den Toleranzbereich um den Aufbau-Bezugspunkt A. Die Abstimmung der federnden Verbindung wird durch die Position der beiden Auflageflächen 6, 7 und durch die Kraft-Weg-Kennlinien der beiden federnden Verbindungen bestimmt. Für die Abstimmung muss gelten

$$a1 \times F1 \approx a2 \times F2.$$

Diese Bedingung, wie auch die Bedingung, dass der Abstand x des Körpervektors vom Aufbau-Bezugspunkt A < d/2 sein soll, gilt für die veränderliche Belastung mit

$$0,25 \times mU < \quad < 0,8 \, mO$$

Diese veränderliche. Belastung wird beim unten erwähnten "simulierten Stehen" vorgenommen.

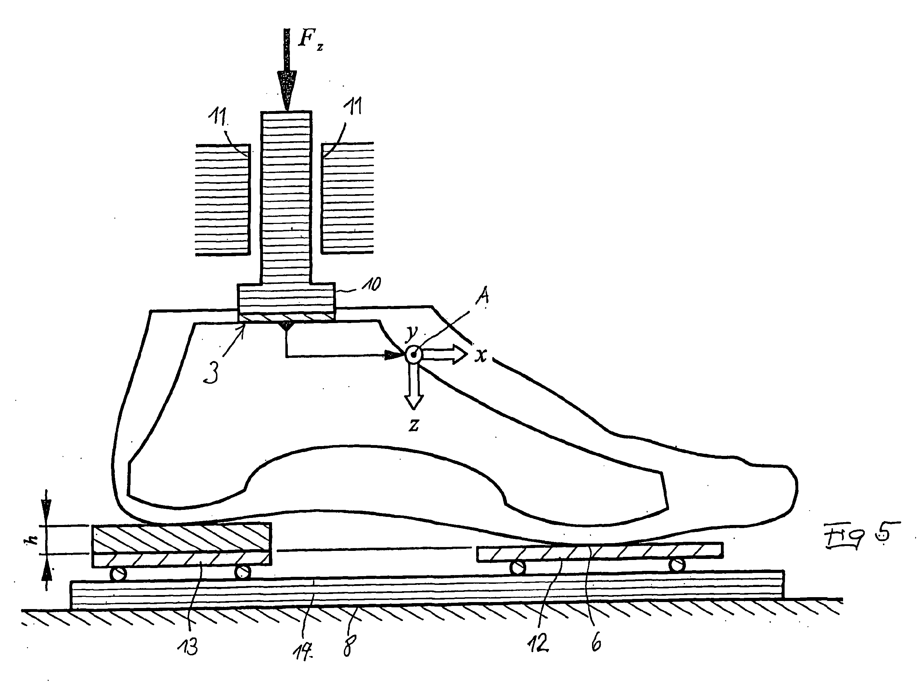

Die Einhaltung der erfindungsgemäßen Bedingung wird mit einem Versuch für ein simuliertes Stehen überprüft, wie er in Figur 5 dargestellt ist. Für den Versuch wird das Unterschenkel-Anschlussteil 4 durch einen eine definierte Krafteinleitung erlaubenden Stempel 10 ersetzt. Der Stempel wird in einer seitlichen Führung 11 so geführt, dass er nur Verschiebungen entlang einer Koordinate Z senkrecht zum Untergrund 8 und keine Rotation durchführen kann. Senkrecht zur Koordinate z spannen die Koordinaten x und y eine Ebene parallel zum Untergrund 8 auf. Der Nullpunkt der Koordinaten x und y wird in den Aufbau-Bezugspunkt A gelegt. Unterhalb der Auflageflächen 6, 7 befinden sich Gleitlager 12, 13. Dadurch sind die Auflageflächen 6, 7 entlang der x-Achse frei beweglich und entlang der y-Achse und der z-Achse gesperrt. Unterhalb der Gleitlager 12, 13 befindet sich eine Kraftmessplatte, mit der sich der aus den Bodenreaktionskräften an den Auflageflächen 6, 7 ergebende resultierende Kraftvektor (Körpervektor) messtechnisch ermittelt werden kann.

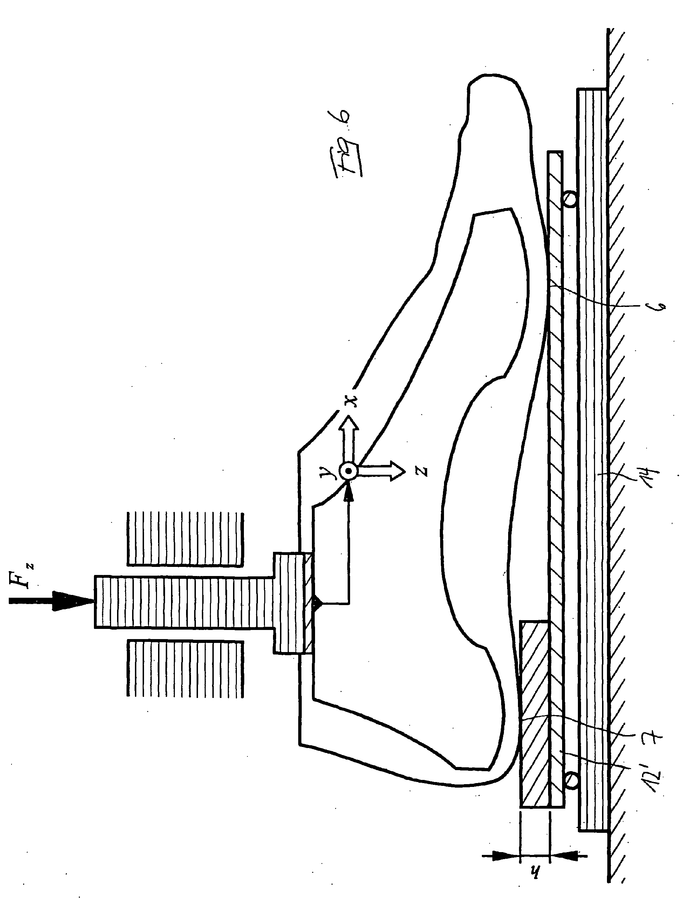

Während in der Anordnung gemäß Figur 5 separate Gleitlager 12, 13 vorgesehen sind, ist in der Modifikation gemäß Figur 6 ein einziges Gleitlager 12' vorgesehen. Folglich treten in der Modifikation wegen der Verformung des künstlichen Fußes unter Last Reibungskräfte entlang der x-Achse auf, wodurch die Messwerte aber nur geringfügig verändert werden.

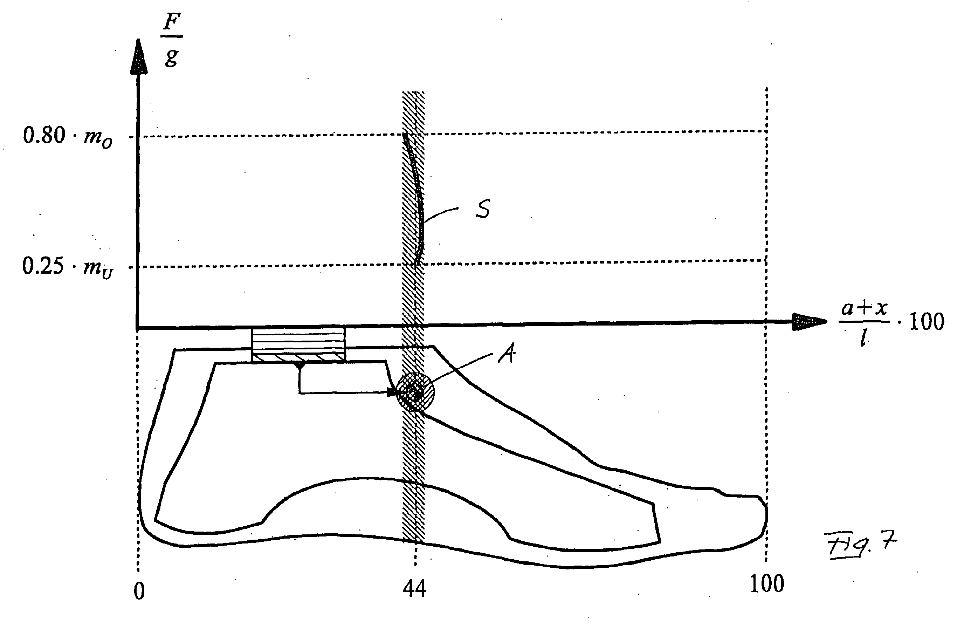

Figur 7 verdeutlicht, dass für die in den Versuchen gemäß Figur 5 oder Figur 6 durchgeführte Gewichtsbelastung von 0,25 % x mU bis 0,8 % x mO der resultierende Kraftvektor der Bodenreaktionskräfte innerhalb einer Schwankungsbreite liegt, die kleiner als der Toleranzbereich d ist. Erfindungsgemäß liegt der Aufbau-Bezugspunkt A, um den die Lage des Körpervektors gemäß der Kurve S schwankt, bei 44 % der Nennlänge I des künstlichen Fußes.

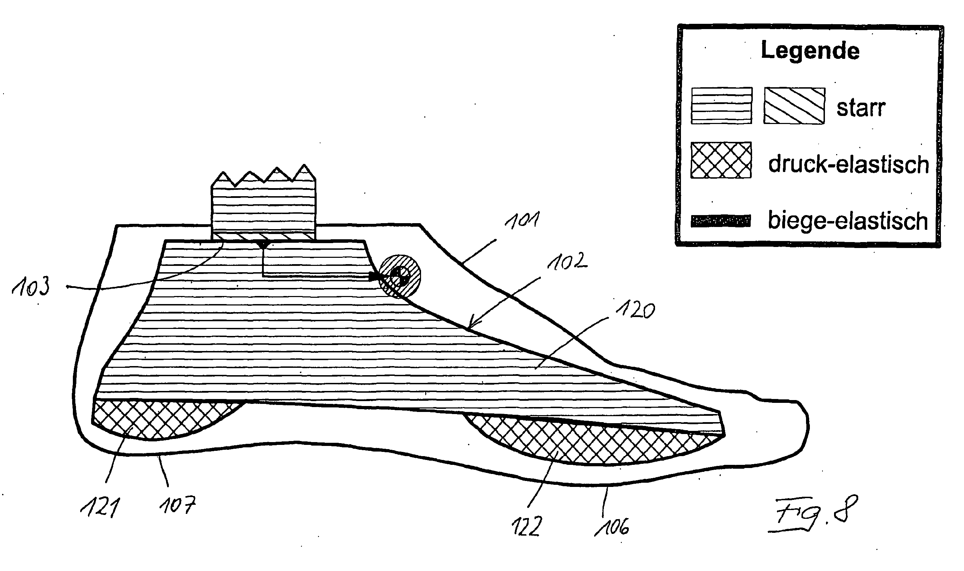

Figur 8 zeigt ein erstes konstruktives Ausführungsbeispiel für einen erfindungsgemäßen künstlichen Fuß. Der Fußeinsatz 102 besteht dabei aus einem starren Kern 120, der die Anschlussfläche 103 ausbildet und sich sowohl in den Ballenbereich als auch in den Fersenbereich des künstlichen Fußes hinein erstreckt. Im Fersenbereich ist an den Kern ein druckelastisches balliges Fersenkissen 121 angesetzt, das die fersenseitige Auflagefläche 107 bestimmt. Die Balligkeit der Auflagefläche 107 kann durch die ballige Ausformung sowohl der Auflagefläche 7 als auch des dahinter befindlichen Fußteils 102 realisiert sein. Die Funktion der Balligkeit der Auflagefläche 107 ergibt sich daraus, dass eine im Wesentlichen linienähnlich Kontaktfläche mit dem Untergrund 8 hergestellt wird, deren Position nicht wesentlich wandert, wenn der künstliche Fuß von einem im Wesentlichen unbelasteten Zustand in einen belasteten Zustand übergeht. Dies ist ersichtlich auch dadurch möglich, dass das Fersenkissen 121 ballig ausgeführt ist, da dieses nur über die kosmetische Hülle 101 mit dem Untergrund 8 bzw. der Standard-Absatzhöhe 9 in Kontakt gerät.

In ähnlicher Form ist bei diesem Ausführungsbeispiel auch die ballenseitige Auflagefläche 106 in ihrer Balligkeit dadurch bestimmt, dass ein druckelastisches Ballenkissen 122 auf der Unterseite des Kerns 120 angesetzt ist. Die Elastizitäten des Fersenkissens 121 und des Ballenkissens 122 sind dabei so aufeinander abgestimmt, dass die erfindungsgemäße Bedingung erfüllt ist, wonach in der Belastung des künstlichen Fußes zwischen 0,25 x mU und 0,8 x mO der resultierende Kraftvektor der Bodenreaktionskräfte innerhalb des Toleranzbereichs d liegen soll.

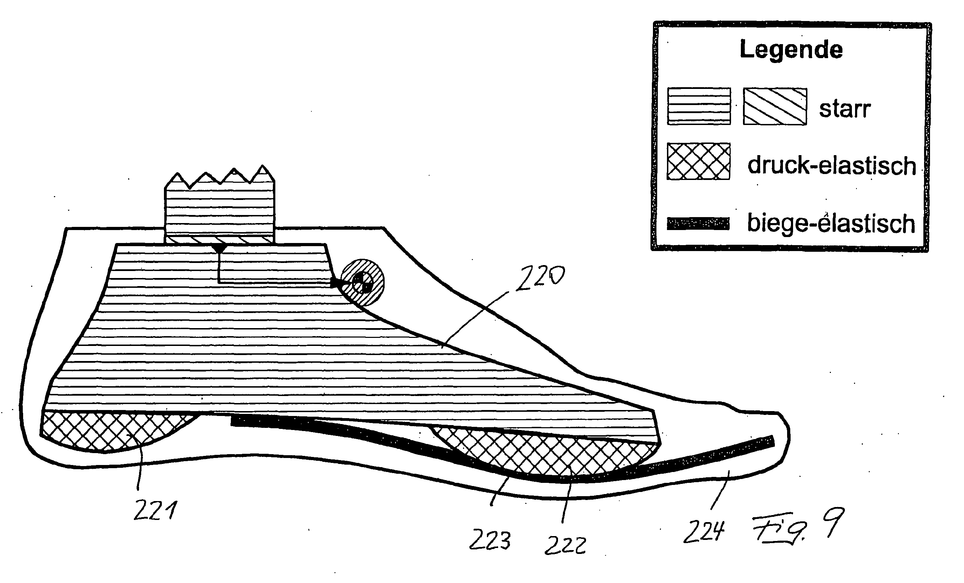

Bei der in Figur 9 dargestellten zweiten Ausführungsform eines erfindungsgemäßen Fußes sind die Konstruktionsmerkmale der ersten Ausführungsform übernommen worden. Zusätzlich ist an die Unterseite des Kerns 220 eine biegeelastische Feder 223 angesetzt worden, die sich mit geringem Abstand von dem Fersenkissen 221 auf der Unterseite des Ballenkissens 222 verlaufend bis in einen Zehenbereich 224 des künstlichen Fußes erstreckt. Die biegeelastische Feder 223, ist vorzugsweise als Blattfeder ausgebildet. Darüber hinaus wird beim Abrollen am Ende der Standphase des Gangzyklus eine verbesserte Zehensteifigkeit in Verbindung mit einem erhöhten Sicherheitsgefühl erreicht.

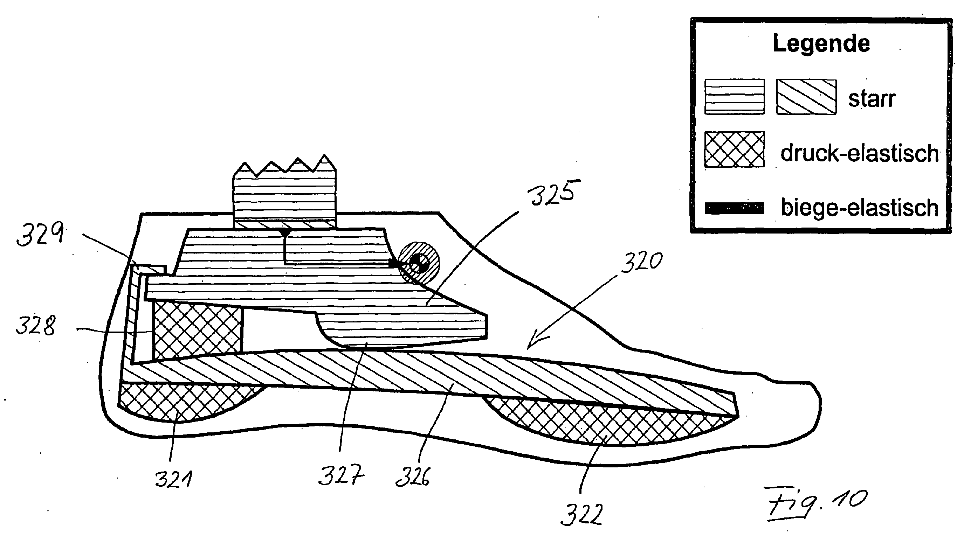

Bei der in Figur 10 dargestellten dritten Ausführungsform eines erfindungsgemäßen Fußes sind - wie bei der in Figur 6 dargestellten ersten Ausführungsform - ein Fersenkissen 321 und ein Ballenkissen 322 vorgesehen. Im Unterschied zu der ersten Ausführungsform besteht der Kern 320 aus zwei starren Teilkernen, einem oberen Teilkern 325 und einen unteren Teilkern 326. Das Fersenkissen 321 und das Ballenkissen 322 befinden sich an der Unterseite des sich über die Länge des künstlichen Fußes erstreckenden unteren Teilkerns 326, während der obere Teilkern mit einer balligen Gelenkfläche 327 auf der Oberseite des unteren Teilkerns 326 aufliegt und gekoppelt ist, sodass zwischen dem oberen Teilkern 325 und dem unteren Teilkern 326 eine Gelenkverbindung besteht, die ein Verschwenken des oberen Teilkerns 325 relativ zum unteren Teilkern 326 ermöglicht. Im Fersenbereich des Fußes ist zwischen der Unterseite des oberen Teilkerns 325 und der Oberseite des unteren Teilkerns 326 ein druckelastisches Kissen 328 angeordnet, das beim Fersenauftritt im Gangzyklus eine federnde Plantarflexion ermöglicht. Um beim Überrollen des Fußes in den Ballenbereich beim Gangzyklus das Dämpferkissen 328 nicht zu überdehnen, übergreift ein Dorsalanschlag 329 des unteren Teilkerns 326 einen Absatz 330 des oberen Teilkerns 325. Die Schwenkbewegung des unteren Teilkerns 326 mit dem Ballenbereich in Richtung Unterschenkel (Dorsalflexion) wird durch den Dorsalanschlag 329 somit begrenzt.

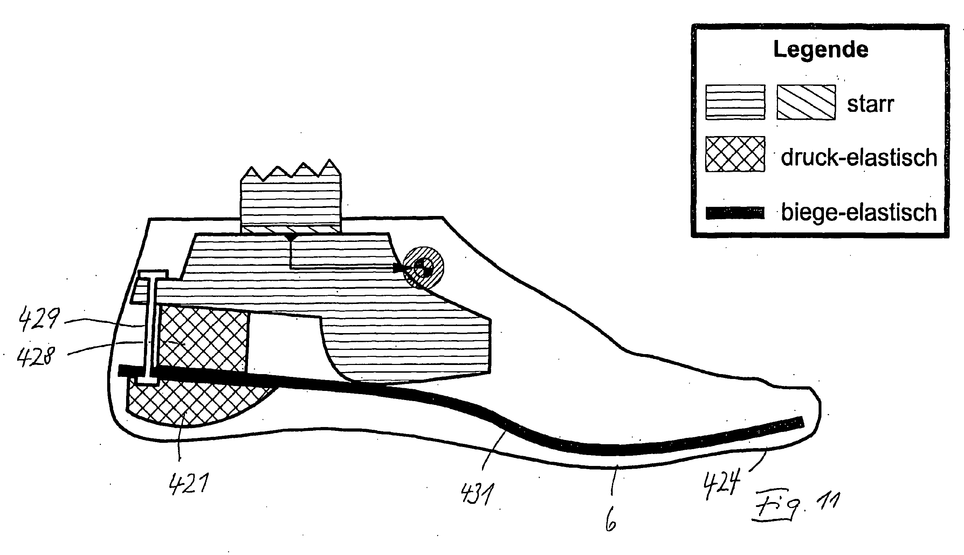

Bei der in Figur 11 dargestellten vierten Ausführungsform ist der untere Teilkern der dritten Ausführungsform zusammen mit dem Ballen kissen durch eine biegeelastische Feder 431 ersetzt, die sich vom Fersenbereich bis zum Zehenbereich 424 des künstlichen Fußes erstreckt. Die biegeelastische Feder 431 ist im Ballenbereich nach unten ballig geformt, um so die ballenseitige Auflagefläche 6 zu bestimmen.

Im Fersenbereich dient die Feder 431 zur Befestigung des Fersenkissens 421 an der Unterseite sowie zur Halterung des Dorsalanschlags 429 und zur Lagerung des Dämpferkissens 428 an der Oberseite der Feder 431.

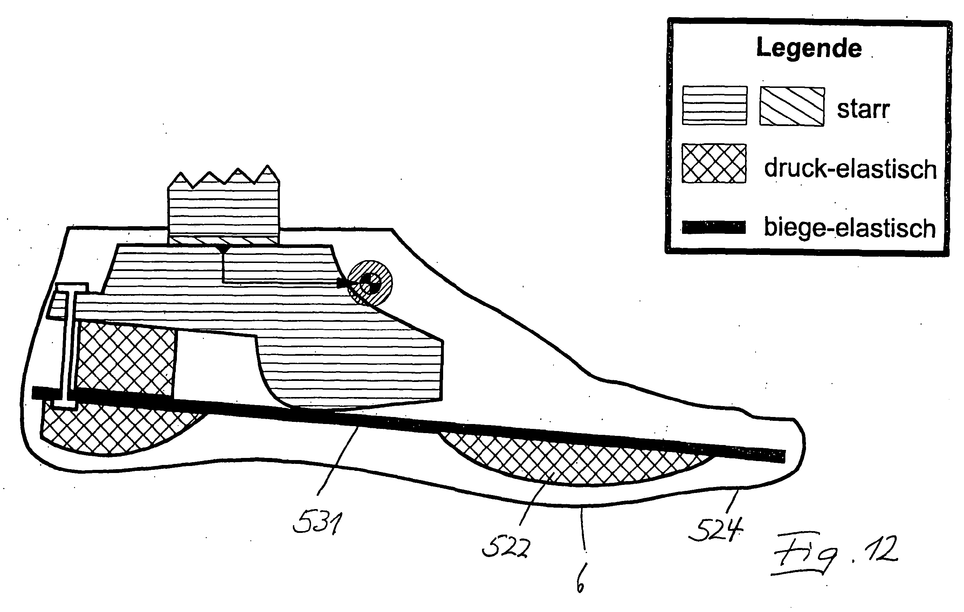

Die in Figur 12 dargestellte fünfte Ausführungsform entspricht im Wesentlichen der vierten Ausführungsform gemäß Figur 9. Allerdings ist die sich über die Fußlänge erstreckende Feder 531 nicht wie in Figur 9 geformt, sondern erstreckt sich im Wesentlichen als gerade Blattfeder in den Zehenbereich 524 des Fußes. Im Ballenbereich ist an der Unterseite der Feder 531 ein Ballenkissen 522 zur Ausbildung der ballenseitigen Auflagefläche 6 befestigt. In dieser Ausführungsform ist es möglich, die Steifigkeit im Zehenbereich 524 durch die Blattfeder unabhängig von der Elastizität im Ballenbereich, die durch das Ballenkissen 522 bestimmt wird, einzustellen.

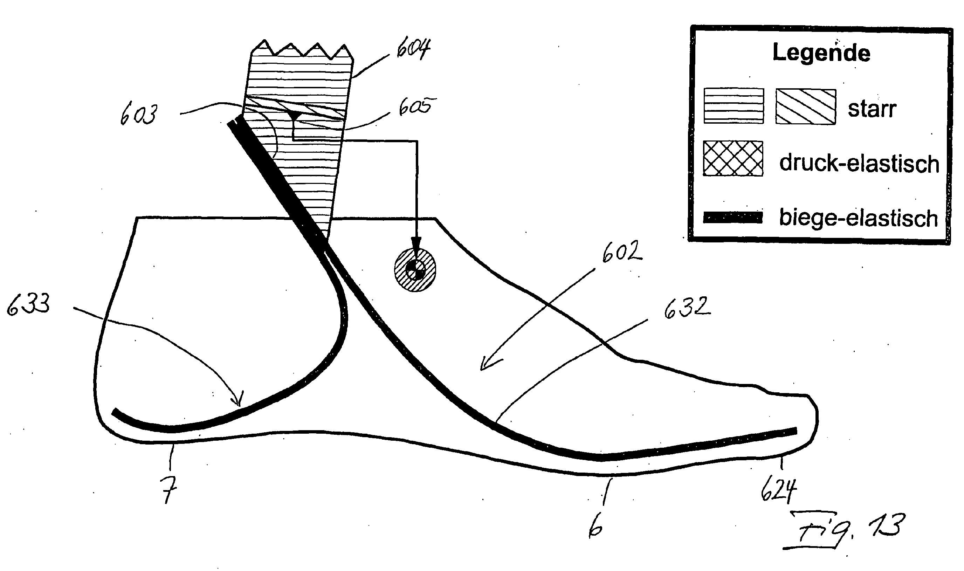

Bei der in Figur 13 dargestellten sechsten Ausführungsform eines erfindungsgemäß hergestellten Fußes besteht das Fußteil 602 ausschließlich aus zwei geformten biegeelastischen Federn, einer Vorfußfeder 632 und einer Fersenfeder 633. Das Unterschenkel-Anschlussteil 604 ist mit einer schrägen Fläche an der entsprechend schräg verlaufenden Anschlussfläche 603 angeschlossen. Die schräge Anschlussfläche 603 wird durch das posteriore Ende der Vorfußfeder 632 gebildet, zu dem ein entsprechendes schräg nach oben gerichtetes Ende der Fersenfeder 633 parallel verläuft, wobei die parallel verlaufenden Abschnitte der Vorfußfeder 632 und der Fersenfeder 633 miteinander verbunden sind. Der virtuelle Krafteinleitungspunkt 605 befindet sich mittig im Unterschenkel-Prothesenteil 604.

Die Vorfußfeder 632 erstreckt sich von der Anschlussfläche 603 mit einer leichten konkaven Wölbung in den Ballenbereich und bestimmt mit der dortigen balligen Ausbildung in Längsrichtung die ballenseitige Auflagefläche 6. Von dort aus erstreckt sich die Vorfußfeder 632 mit einem etwa gradlinig verlaufenden Ende in den Zehenbereich 624 hinein.

Die Fersenfeder 633 erstreckt sich von der Anlagefläche 603 mit einer nach hinten gerichteten Krümmung in den Fersenbereich hinein und bildet mit der entsprechenden balligen Ausformung in Längsrichtung die fersenseitige Auflagefläche 7 aus.

Die Federhärten der Vorfußfeder 632 und der Fersenfeder 633 sind so aufeinander abgestimmt, dass das durch die Federkombination gebildete Fußteil 602 die erfindungsgemäße Bedingung für die konstante Lage des Schwerpunkts der Bodenreaktionskräfte erfüllt.

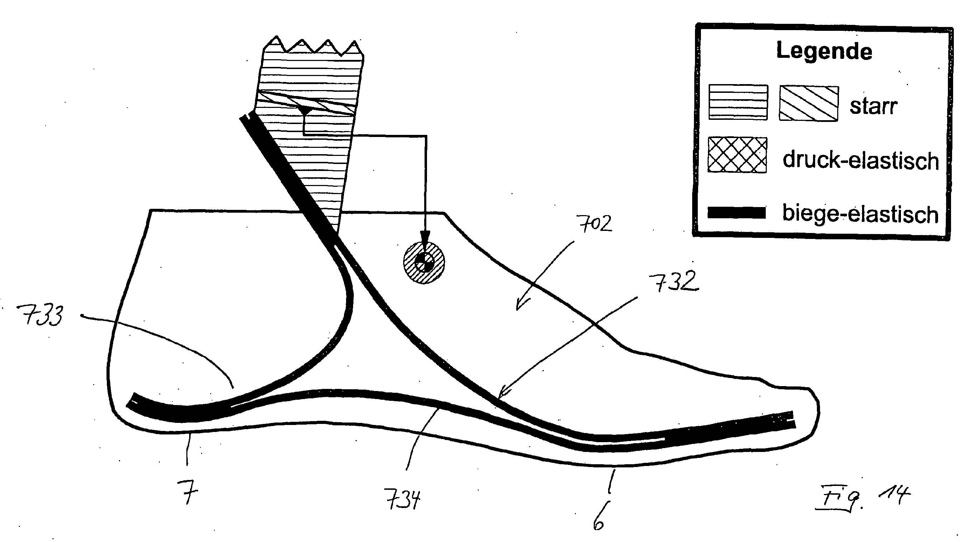

Die in Figur 14 dargestellte siebte Ausführungsform eines erfindungsgemäß hergestellten Fußes entspricht der in Figur 11 dargestellten sechsten Ausführungsform, weist jedoch zusätzlich eine den Fersenbereich der Fersenfeder 733 und den vorderen Teil der Vorfußfeder 732 verbindende Sohlenfeder 734 auf.

Die Sohlenfeder ist so geformt, dass sie sich der balligen Ausbildung der Fersenfeder 733 im Bereich der fersenseitigen Auflagefläche 7 und der balligen Ausbildung der Vorfußfeder im Bereich der ballenseitigen Auflagefläche 6 anpasst. Dazwischen ist die Sohlenfeder 734 konvex gekrümmt, um die Auflageflächen 6, 7 brückenartig miteinander zu verbinden. Die zusätzliche Sohlenfeder 734 sorgt für eine gleichmäßigere Verteilung der Deformationsenergie auf die Vorfußfeder 732 und die Fersenfeder 733 bei der Belastung im Gangzyklus. Auch hier erfolgt die Abstimmung der Federkombination des Fußteils 702 so, dass die erfindungsgemäße konstante Lage des resultierenden Kraftvektors der Bodenreaktionskräfte (Körpervektor) eingehalten wird.

**Patentansprüche**

1. Verfahren zur Herstellung eines künstlichen Fußes, der in einer Längsrichtung eine mediale Ebene (M) aufweist, in der eine Nenn-Fußlänge (I) einen Abstand von einer Ferse und einer Fußspitze eines von dem künstlichen Fuß ersetzten natürlichen Fußes definiert ist, und der mit einem oberseitigen Anschlussstück (4) zur momentensteifen Verbindung eines sich im Wesentlichen über die Fußlänge (I) erstreckenden Fußeinsatzes (2) ausgebildet ist, der - über die Länge (I) gesehen - an zwei Auflageflächen (6, 7) aufliegt, von denen sich eine erste fersenseitige Auflagefläche (7) im Fersenbereich und eine zweiten ballenseitige Auflagefläche (6) im Ballenbereich befindet und der so ausgebildet ist, dass das Anschlussteil (4) über federnde Verbindungen mit den Auflageflächen (6, 7) des Fußteils verbunden ist, wobei die balligen Auflageflächen (6, 7) zu einer angenähert linienförmigen Auflagefläche auf einem Untergrund (8) ausgebildet sind, **gekennzeichnet durch** die Verfahrensschritte

a) Positionierung eines Körpervektors als resultierenden Kraftvektor der an den Auflageflächen (6, 7) entstehenden Bodenreaktionskräften im ruhenden Stand in einem Bereich zwischen 40 % und 48 % der Nenn-Fußlänge (I), von der Ferse aus gemessen,

b) Belastung des Fußes mit Gewichtsbelastungen zwischen 25 % des minimal zulässigen und 80 % des maximal zulässigen Körpergewichts und

c) Abstimmung der federnden Verbindungen zwischen dem Anschlussteil (4) und den Auflageflächen (6, 7) derart, dass sich bei der Belastung gemäß Verfahrensschritt b) der Körpervektor um weniger als ± 4 % der Nenn-Fußlänge (I) verschiebt.

2. Verfahren nach Anspruch 1, **dadurch gekennzeichnet, dass** die Positionierung des Körpervektors bei den Gewichtsbelastungen gemäß Verfahrensschritt d) in einem Abstandsbereich zwischen 42 % und 46 % der Nenn-Fußlänge (I) verbleibt.

3. Verfahren nach Anspruch 1 oder 2, **dadurch gekennzeichnet, dass** der Fußeinsatz (2) mit einem Kern (120) ausgebildet und dem Auflageflächen (6, 7) über elastische Stücke (121, 122) verbunden wird.

4. Verfahren nach Anspruch 3, **dadurch gekennzeichnet, dass** die balligen Auflageflächen (6, 7) mit den elastischen Stücken (120, 121) aus elastisch komprimierbarem Material bestimmt werden.

5. Verfahren nach Anspruch 4, **dadurch gekennzeichnet, dass** als elastische Stücke (120, 121) Federn verwendet werden.

6. Verfahren nach einem der Ansprüche 1 bis 5, **dadurch gekennzeichnet, dass** das Fußteil (2) mit einer sich über die Länge des Fußteils erstreckenden Blattfeder (223) ausgebildet wird, deren Federhärte zum Zehenbereich abnimmt.

7. Verfahren nach einem der Ansprüche 1 bis 6 zur Herstellung eines künstlichen Fußes, dessen Fußeinsatz (2) ein Gelenk enthält, **dadurch gekennzeichnet, dass** der Drehpunkt des Gelenks in die konstruktive Schwerpunktslinie im ruhenden Stand des Prothesenträgers gelegt wird und bei Belastung ohne Längenänderung in der Schwerpunktslinie eine elastisch bedämpfte Drehung des Anschlussteils (4) relativ zur fersenseitigen Auflagefläche (7) ermöglicht.

8. Verfahren nach Anspruch 7, **dadurch gekennzeichnet, dass** zwischen der fersenseitigen Auflagefläche (7) und dem Anschlussteil (4) eine komprimierbare Feder (328) eingesetzt wird.

9. Verfahren nach Anspruch 8, **dadurch gekennzeichnet, dass** die Drehbewegung in Richtung Expansion der Feder (328) begrenzt wird.

10. Verfahren nach Anspruch 1 oder 2, **dadurch gekennzeichnet, dass** der Fußeinsatz (2) mit einer Federkombination gebildet wird, die eine zur fersenseitigen Auflagefläche (7) verlaufende Fersenfeder (633) und eine zur ballenseitigen Auflagefläche (6) verlaufende Vorfußfeder (632) aufweist und dass die Federn am Anschlussteil (4) miteinander verbunden werden.

11. Verfahren nach Anspruch 10, **dadurch gekennzeichnet, dass** die Feder (632, 633) mit einer sich über die Länge des Fußteils (2) erstreckenden weiteren Feder (734) bodenseitig miteinander verbunden werden.

12. Verfahren nach Anspruch 10 oder 11, **dadurch gekennzeichnet, dass** als Feder (632, 633, 734) Blattfedern verwendet werden.

13. Künstlicher Fuß, herstellbar nach dem Verfahren nach einem der Ansprüche 1 bis 12.

**Claims**

1. A method for producing an artificial foot which, in a longitudinal direction, has a medial plane (M) in which a nominal foot length (1) is defined as a distance from a heel to a foot tip of a natural foot replaced by the artificial foot, and which is designed with a top connector piece (4) for the torsionally rigid connection of a foot insert (2) extending substantially along the length (1) of the foot, and which, seen along the length (1), bears on two contact surfaces (6, 7), of which a first heel-side contact surface (7) is located in the heel area and a second ball-side contact surface (6) is located in the ball area, and which is designed such that the connector part (4) is connected to the contact surfaces (6, 7) of the foot part by spring connections, wherein the curved contact surfaces (6, 7) are designed for an approximately linear contact surface on a support (8), **characterized by** the following method steps:

a) positioning a body vector as resulting force vector of the ground reaction forces occurring on the contact surfaces (6, 7) in the rest state in a range between 40% and 48% of the nominal foot length (1), measured from the heel,

b) loading the foot with weight loads between 25% of the minimal permissible and 80% of the maximum permissible body weight, and
c)adapting the spring connections between the connector part (4) and the contact surfaces (6, 7) in such a way that, during the loading according to method step b), the body vector shifts by less than ± 4% of the nominal foot length (1).

2. The method as claimed in claim 1, **characterized in that** the positioning of the body vector during the weight loads according to method step d) remains in a distance range between 42% and 46% of the nominal foot length (1).

3. The method as claimed in claim 1 or 2, **characterized in that** the foot insert (2) is designed with a core (120) and is connected to the contact surfaces (6, 7) via elastic pieces (121, 122).

4. The method as claimed in claim 3, **characterized in that** the curved contact surfaces (6, 7) with the elastic pieces (120, 121) are determined from elastically compressible material.

5. The method as claimed in claim 4, **characterized in that** springs are used as elastic pieces (120, 121).

6. The method as claimed in one of claims 1 through 5, **characterized in that** the foot part (2) is designed with a leaf spring (223) which extends along the length of the foot part and whose spring hardness decreases toward the toe area.

7. The method as claimed in one of claims 1 through 6 for producing an artificial foot whose foot insert (2) contains a joint, **characterized in that** the pivot point of the joint is placed in the structural line of gravity in the rest state of the prosthesis wearer and, upon loading, permits an elastically damped rotation of the connector part (4) relative to the heel-side contact surface (7) without a change of length in the line of gravity.

8. The method as claimed in claim 7, **characterized in that** a compressible spring (328) is fitted between the heel-side contact surface (7) and the connector part (4).

9. The method as claimed in claim 8, **characterized in that** the rotation movement is limited in the direction of expansion of the spring (328).

10. The method as claimed in claim 1 or 2, **characterized in that** the foot insert (2) is formed with a spring combination that has a heel spring (633) extending to the heel-side contact surface (7) and a forefoot spring (632) extending to the ball-side contact surface (6), and **in that** the springs are connected to each other on the connector part (4).

11. The method as claimed in claim 10, **characterized in that** the springs (632, 633) are connected to each other at the ground side via a further spring (734), which extends along the length of the foot part (2).

12. The method as claimed in claim 10 or 11, **characterized in that** leaf springs are used as springs (632, 633, 734).

13. An artificial foot that can be produced by the method as claimed in one of claims 1 through 12.

**Revendications**

1. Procédé pour la fabrication d'un pied artificiel qui présente en direction longitudinale un plan médian (M) dans lequel est définie une longueur nominale (1) du pied, une distance depuis un talon et depuis une pointe d'un pied naturel remplacé par le pied artificiel, et qui est réalisé avec une pièce de raccordement (4) du côté supérieur pour la liaison, rigide vis-à-vis des couples, d'un insert de pied (2) s'étendant sensiblement sur la longueur (1) du pied, ledit insert reposant, vu sur la longueur (1), sur deux surfaces d'appui (6, 7), parmi lesquelles une première surface d'appui côté talon (7) se trouve dans la région du talon et une seconde surface d'appui (6) côté coussinet ce trouve dans la région du coussinet, et étant ainsi réalisé que la pièce de raccordement (4) est reliée avec les surfaces d'appui (6, 7) de la partie de pied via des liaisons à effet ressort, et les surfaces d'appui côté coussinet (6, 7) sont réalisées sur une base sous-jacente (8) en formant une surface d'appui de forme approximativement linéaire,
**caractérisé par** les étapes de procédé consistant à

a) positionner un vecteur corporel à titre de vecteur de force résultant des forces de réaction du sol apparaissant au niveau des surfaces d'appui (6, 7) en station debout au repos dans une plage entre 40 % et 48 % de la longueur nominale (1) du pied, mesurée depuis le talon,
b) charger le pied avec des charges pondérales entre 25 % du poids corporel minimum admissible et 80 % du poids corporel maximum admissible, et
c) ajuster les liaisons à effet ressort entre la pièce de raccordement (4) et les surfaces d'appui (6, 7) de telle façon que, lors de la mise en charge selon l'étape b) le vecteur corporel se déplace de moins de ± 4 % de la longueur nominale (1) du pied.

2. Procédé selon la revendication 1, **caractérisé en ce que** le positionnement du vecteur corporel, sous les charges pondérales selon l'étape b), demeure dans une zone de distance entre 42 % et 46 % de la longueur nominale (1) du pied.

3. Procédé selon la revendication 1 ou 2, **caractérisé en ce que** l'insert de pied (2) est réalisé avec un noyau (120) et est relié aux surfaces d'appui (6, 7) via des pièces élastiques (121, 122).

4. Procédé selon la revendication 3, **caractérisé en ce que** les surfaces d'appui (6, 7) côté coussinet avec les pièces élastiques (120, 121) sont réalisées en matériau susceptible d'être élastiquement comprimé.

5. Procédé selon la revendication 4, **caractérisé en ce que** l'on utilise des ressorts à titre de pièces élastiques (120, 121).

6. Procédé selon l'une des revendications 1 à 5, **caractérisé en ce que** la partie de pied (2) est réalisée avec un ressort à lame (223) qui s'étend sur la longueur de la partie de pied et dont la raideur élastique diminue vers la région des orteils.

7. Procédé selon l'une des revendications 1 à 6 pour la fabrication d'un pied artificiel dont l'insert de pied (2) contient une articulation, **caractérisé en ce que** le centre de rotation de l'articulation est placé sur la ligne de gravité structurelle dans la station debout au repos du porteur de prothèse et permet, en cas de charge sans modification de longueur dans la ligne de gravité, une rotation élastique amortie de la pièce de raccordement (4) par rapport à la surface d'appui (7) côté talon.

8. Procédé selon la revendication 7, **caractérisé en ce qu'**un ressort susceptible d'être comprimé (328) est mis en place entre la surface d'appui (7) côté talon et la pièce de raccordement (4).

9. Procédé selon la revendication 8, **caractérisé en ce que** le mouvement de rotation est limité dans la direction de l'expansion du ressort (328).

10. Procédé selon la revendication 1 ou 2, **caractérisé en ce que** l'insert de pied (2) est formé avec une combinaison de ressorts qui comprend un ressort de talon (633) s'étendant vers la surface d'appui (7) côté talon et un ressort d'avant-pied (632) s'étendant vers la surface d'appui (6) côté coussinet, et **en ce que** les ressorts sont reliés l'un à l'autre au niveau de la pièce de raccordement (4).

11. Procédé selon la revendication 10, **caractérisé en ce que** les ressorts (632, 633) sont reliés l'un à l'autre du côté du sol avec un autre ressort (734) qui s'étend sur la longueur de la partie de pied (2).

12. Procédé selon la revendication 10 ou 11, **caractérisé en ce que** l'on utilise des ressorts à lame à titre de ressorts (632, 633, 734).

13. Pied artificiel, susceptible d'être fabriqué selon le procédé de l'une des revendications 1 à 12.

Fig.1

$$a = 0.44 \cdot l$$
$$d = 2 \cdot 0.04 \cdot l$$

Fig. 2

$$\frac{a_1}{a_2} = \frac{l_1}{l_2} = \frac{b_1}{b_2}$$

Fig. 3

$$-\left(\vec{F_1}+\vec{F_2}\right) = \vec{F}$$

Fig. 4

EP 2 355 753 B1

Fig 5

Fig.6

Fig. 7

Fig. 8

Fig. 9

Legende

starr

druck-elastisch

biege-elastisch

Fig. 10

320

325

322

326

327

321

329

328

Legende

starr

druck-elastisch

biege-elastisch

Fig. 11

424

6

431

421

429

428

Legende

starr

druck-elastisch

biege-elastisch

531

522

6

524

Fig. 12

EP 2 355 753 B1

**Legende**

| | | |
|---|---|---|
| ▦ | ▨ | starr |
| ▩ | | druck-elastisch |
| ▬ | | biege-elastisch |

604

603

605

602

632

633

7

6

624

Fig. 13

EP 2 355 753 B1

Fig. 14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20030144745 A1
- GB 2433443 A